# EUROPEAN PATENT SPECIFICATION

(11) **EP 1 033 938 B1**
(45) Date of publication and mention of the grant of the patent: **28.02.2007**
(21) Application number: 98959595.4
(22) Date of filing: 24.11.1998
(51) Int. Cl.: A61B 17/00, A61B 17/04

(54) **SUTURE FASTENING DEVICE**
BEFESTIGUNGSVORRICHTUNG FÜR NÄHMATERIAL
DISPOSITIF D'ATTACHE DE SUTURES

(30) Priority: 24.11.1997 US 66879 P
(43) Date of publication of application: 13.09.2000
(73) Proprietor: Axya Medical Inc., Beverly, MA 01915 (US)
(72) Inventor: EGAN, Thomas, D., Marblehead, MA 01945 (US); STREETER, Richard, B., Winchester Massachusetts 01890 (US)
(74) Representative: Greenwood, John David
(86) International application number: PCT/US1998/025143
(87) International publication number: WO 1999/026542

(56) References cited:
- US-A- 3 513 848
- US-A- 5 383 883
- US-A- 5 417 700

## Description

### FIELD OF THE INVENTION

The present invention relates generally to closing or joining openings or wounds, tying off vessels, and attaching anatomical and foreign structures in human and animal tissue and the like, and more particularly to devices for suturing, ligating and attaching structures, including hand held devices with specific application and utility in hard to reach surgical situations.

### BACKGROUND OF THE INVENTION

U.S. Patent Nos. 3,513,848 to Winston et al., 4,662,068 to Polonsky, and 5,383,883 to Wilk et al. describe joining surgical suture by welding or fusing strands together. The present invention includes significant advances over the prior art.

First among these is control of critical parameters affecting the strength properties of the weld. Principal among these is the force with which the ultrasonic element is brought to bear against the material to be welded. Winston et al. describe a device where this force is provided by the user through pressure applied by the thumb and forefingers. This method of pressure control is subject to substantial variation from weld to weld and from user to user. Similarly, Polonsky and Wilk et al. describe scissors handled instruments with pivoting jaws that apply pressure proportional to the hand pressure of the user. Another critical control parameter is the amount of energy imparted to the weld. Too much energy imparted to the weld would result in complete melting of the weld region, resulting in an amorphous mass of greatly reduced strength. Similarly, too little energy would result in a reduced or absent weld area, also resulting in reduced weld strength. Here again Winston et al., Polonsky and Wilk et al. provide only for timed energy input at the discretion of the user and therefore subject to substantial variation.

Another advance of the present invention over the prior art is the inclusion of means for controlling the morphology of the material in the weld region to produce welds of significantly greater strength than those produced by the apparatus and method described by Winston et al., Polonsky and Wilk et al. Our experience has shown that welds of superior strength are created when the following conditions are satisfied: 1) The weld is configured as a lap weld with load applied from opposite ends of the long axis of the weld (i.e., loaded in shear, not in peel) 2) the area of the welded region is large, 3) a substantial portion of the suture on either side of the weld area has not been subjected to sufficient heat to reduce the tensile strength of the material, and 4) there is a gradual transition from the stressed full cross section of material outside the weld region to the point where the maximum proportion of cross section has been sacrificed to melting to form the weld. Polonsky and Wilk et al. describe the exiting ends of the suture being welded in an orientation such that loads placed upon the suture would subject the weld to peeling stress. Winston et al. describe welds that would load in peel and others in shear, however those loaded in shear are formed by crossing the suture, a practice resulting in weld areas limited to the small region where the segments overlap in a crosswise fashion. Similarly, Polonsky and Wilk et al. refer to twisting the suture, yielding the same end result as crossing the strands. In order to preserve a non-melted (and therefore non-weakened) portion of the material cross section outside of the weld area, melting must be localized to the region where the overlapping segments of suture abut each other. This condition is best accomplished in ultrasonic welding a lap joint when the two segments to be joined are acoustically coupled to structures that vibrate relative to each other. Experience has shown that acoustic coupling is best accomplished by large areas of contact with the vibrating and non-vibrating or counter-vibrating structures. Winston et al describe "welding tips having a small area of contact" and Winston et al., Polonsky and Wilk et al. show flat welding surfaces in contact with round suture, a situation resulting in thin, line contact of a small area. The present invention includes surfaces which conform to the suture to maximize acoustic coupling.

A further advance over Winston et al., Polonsky and Wilk et al. is the inclusion of means to facilitate loading suture into the device in an orientation conducive to optimum weld characteristics in vivo. Winston et al., Polonsky and Wilk et al. require the user to cross or twist the suture and place it into a slot in the device. This maneuver is difficult to perform in laparoscopic surgery and would likely require withdrawing the suture ends and the instrument from the body cavity for suture loading. In the present invention, suture ends need only be held under slight tension and a simple grab-twist-grab motion employed to load the suture. Another advantage of the present invention over Winston et al is its means for releasing the finished stitch from the device without compressing tissue inside the stitch or stretching the suture material. The present invention also represents an advance over Winston et al. by including a welding apparatus structure constructed with an elongated shaft suitable for use in minimally invasive surgery (MIS) where suturing must be performed internally through a small incision, or where the instrument must be introduced through a tubular structure. Further advances over Winston et al. and Wilk et al. include a means for cutting the exiting ends of the suture.

It is an object of the present invention to provide an instrument for joining lengths of polymer suture material in areas of difficult or limited access, such as in minimally invasive surgery (MIS).

It is a further object of the present invention to provide an instrument with means to provide a stitch for tissue approximation, wound closure, ligating, attachment or suture anchoring functions.

It is a further object of the present invention to provide an instrument with means to provide an attached structure with exiting strands to be used for further tissue approximation, wound closure, ligating, attachment or suture anchoring functions.

It is a further object of the present invention to provide an instrument with means to facilitate loading suture material strands in areas of difficult or limited surgical access.

It is a further object of the present invention to provide an instrument with means for releasing joined surgical suture loops without stretching the loop or compressing the tissue material within the loop.

It is a further object of the present invention to provide an instrument with means for facilitating sliding passage of at least one of the suture material strands exiting a loop to facilitate tensioning of the loop.

It is a further object of the present invention to provide an instrument with means for clamping or securing one suture material strand exiting a loop to facilitate tensioning of the loop by pulling on the remaining, non-secured strand.

It is a further object of the present invention to provide an instrument with means for cutting one or more of the strands of suture material exiting the tensioned, joined, finished loop.

It is a further object of the present invention to provide an instrument with means for shielding the tissue to be sutured from direct contact with the vibrating ultrasonic member, thereby protecting the tissue from injury.

It is a further object of the present invention to provide an instrument with means for removing and replacing tissue contacting portions of the device to assist in maintaining sterility through disassembly for cleaning, or disposal and replacement of the tissue contaminated elements.

It is a further object of the present invention to provide an instrument with means for controlling the force with which the segments of material to be welded are held together before, during and after the welding process.

It is a further object of the present invention to provide an instrument with means for controlling the total weld energy imparted to the segments to be welded.

### SUMMARY OF THE INVENTION

According to one aspect of the invention, there is provided a device for forming a welded joint in an elongated material used to ligate living tissue. The device comprises the features defined in claim 1.

In a preferred embodiment of the device, the anvil is a split structure adapted for transverse movement relative to the horn so as to effect closure of the anvil about a portion of the horn. The anvil can be split either symmetrically or asymmetrically.

In a preferred embodiment, the segments are overlapped so that they share a common directionality in the joint region.

At least one of the horn and the anvil is contoured for maximum contact with the elongated material.

The device can further comprise a severing element on at least one of the horn and the anvil, which is adapted to cause localized melting of a suture segment in proximity thereto.

In a preferred embodiment, the element for actuating the horn and anvil for movement relative to one another includes a trigger on the handle and connected to the horn and anvil, a first spring disposed within the handle and coupled with the horn for biasing the horn toward the distal end of the shaft, and a second spring disposed within the tubular shaft for biasing the anvil away from the distal end of the shaft.

The device can further include an element detecting a characteristic of at least one of the welding structure and the welded joint after actuation of the horn and anvil, and an element for controlling the delivery of energy to the welding structure when the characteristic reaches a predetermined value. In a preferred embodiment, this latter element comprises a switch for making or breaking an electrical circuit when the characteristic reaches a predetermined value.

In one embodiment, the characteristic to be detected is displacement of the horn relative to the anvil after actuation of the horn and anvil. In this embodiment, the detecting element comprises a linear displacement gauge which may be located on the horn or the anvil or on a structure which is stationary relative to the horn.

In an alternate embodiment, the characteristic to be detected is time elapsed after actuation of the horn and anvil. In this embodiment, the detecting element comprises a clock.

In still another embodiment, the characteristic to be detected is force exerted by the horn on the segments after actuation of the horn and anvil and during welding of the segments. In this embodiment, the detecting element comprises a transducer.

The power supply preferably comprises an ultrasonic signal generator and an ultrasonic transducer coupled to the horn of the welding structure.

These and other features of the invention will be more fully appreciated with reference to the following detailed description which is to be read in conjunction with the attached drawings.

### BRIEF DESCRIPTION OF THE DRAWINGS

Fig. 1a is an isometric view of the device, according to a preferred embodiment,
Fig. 1b is a detail isometric view of the tip of the device,
Fig. 2a is section view of the device; anvils closed, horn retracted,
Fig. 2b is section view of the device; anvils open, horn retracted,
Fig. 2c is section view of the device; anvils closed, horn engaged,
Fig. 3 is a detail isometric view of the tip with exterior components removed,
Fig 4a is a section view of the tip of the device in the x-z plane; anvils open, horn retracted,
Fig 4b is a section view of the tip of the device in the y-z plane; anvils open, horn retracted,
Fig 5a is a section view of the tip of the device in the x-z plane; anvils closed, horn retracted,
Fig 5b is a section view of the tip of the device in the y-z plane; anvils closed, horn retracted,
Fig 6a is a section view of the tip of the device in the x-z plane; anvils closed, horn engaged,
Fig 6b is a section view of the tip of the device in the y-z plane; anvils closed, horn engaged,
Fig 6c is a detail section view showing the trim mechanism,
Fig 6d is a view of the completed, released stitch,
Fig. 7a through 7f are views of sequential steps used in one type of suturing performed according to the present invention.

### DETAILED DESCRIPTION OF THE DRAWINGS

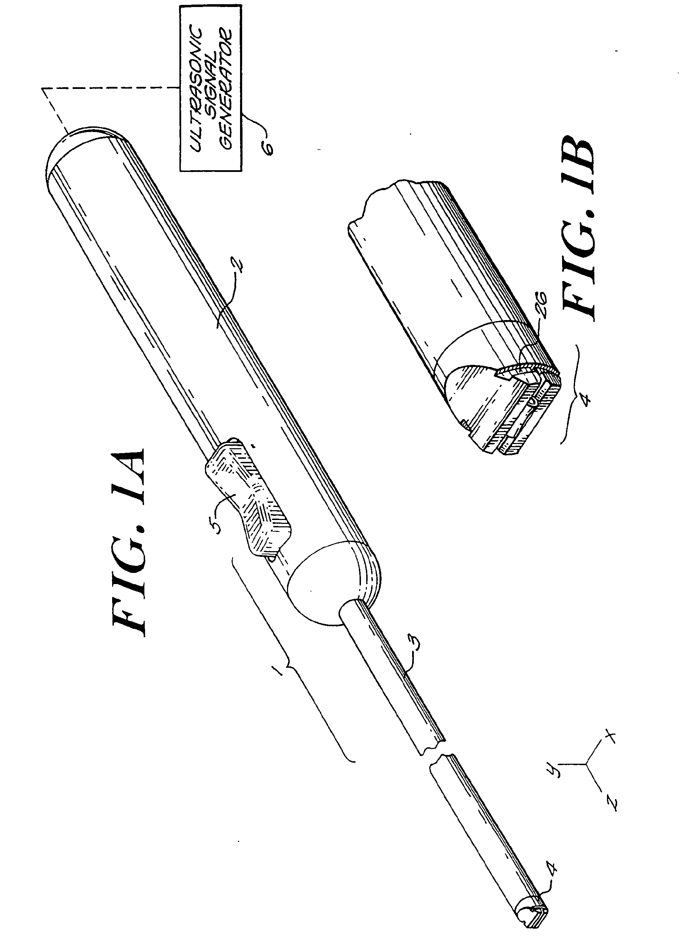

Fig. 1a shows a preferred embodiment of the present invention is expressed as a suture joining device **1** comprising a handle portion **2,** a shaft portion **3** of a length and diameter appropriate for introduction through a laparoscopic trocar cannula, and a slotted tip portion **4** at the distal end of the shaft. In a preferred embodiment a three position mechanical actuator **5** is included to control the suture capture, weld and release functions of the device. Other embodiments employ motors, solenoids, pneumatics and other actuating technology known to the art to control the mechanical elements. One preferred embodiment includes an ultrasonic signal generator **6** connected to the handle portion **2** by an electrical cable. Other preferred embodiments provide a self contained ultrasonic signal generator and battery or other power source in the handle portion **2** itself.

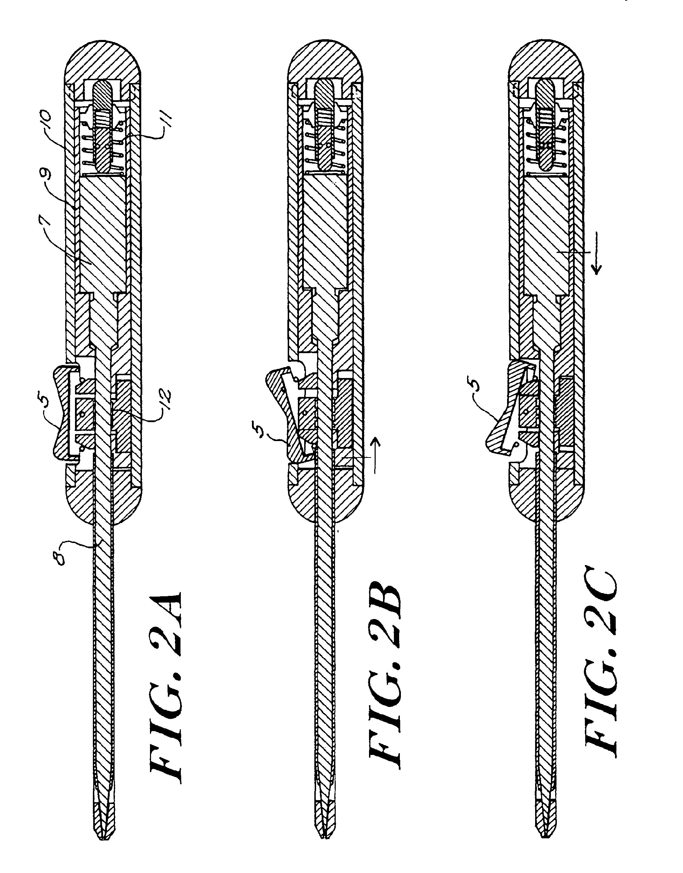

Referring to Fig 2a, the handle houses an ultrasonic transducer **7** coupled to a tuned ultrasonic horn **8.** The transducer and horn are free to slide axially inside a housing **9** which in turn is free to slide axially inside the handle shell **10.** Spring **11** biases the transducer **7** and horn **8** in a distal direction within the housing **9.** The force applied by spring **11** is equal to the optimum horn force required to achieve peak weld properties when compressed to the weld position. Spring **12** biases the housing **9** and its contents in a proximal direction. Actuator **5** is constructed to perform the dual function of opening the slotted tip **4** allowing the instrument to receive suture strands (actuator **5** position shown in Fig. 2b) and to initiate the welding process (actuator **5** position shown in Fig. 2c).

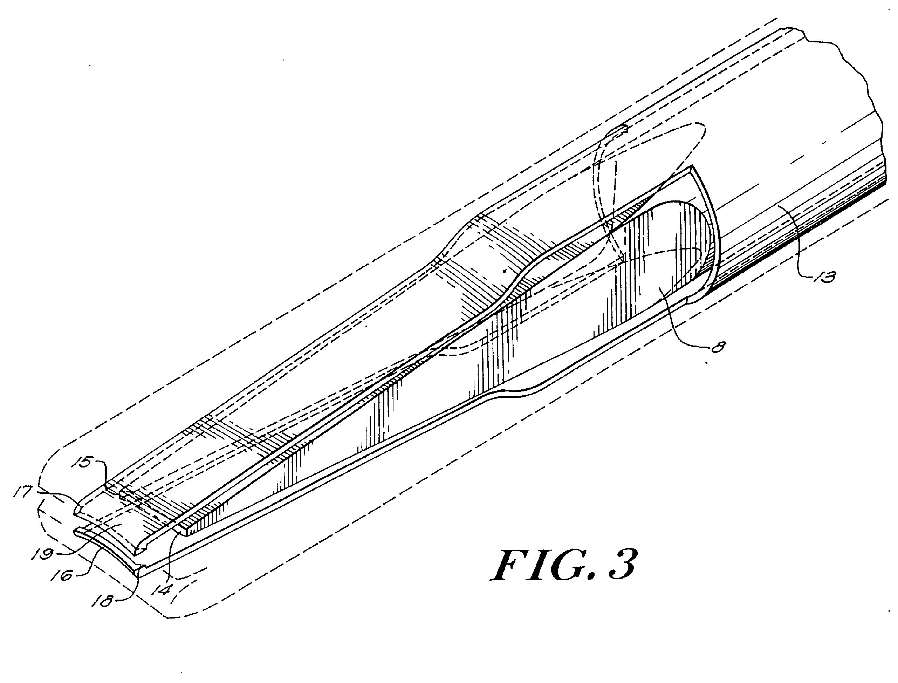

Fig 3 shows the interior of the distal end of the device with the tubular shaft **3** and the slotted tip **4** removed. Here we see the distal end of the horn **8** and a tubular inner shaft **13.** The horn **8** terminates in a radiused conforming surface **14** designed to conform to the shape of the suture to maximize contact area where limited by the width of the horn **8** and half the circumference of the suture. Other embodiments employ V-shaped, faceted or non-circular contours to increase contact area beyond that attainable by a flat surface. In one embodiment, a protruding edge **15** is present at one end of the radiused conforming surface **14.** The tubular inner shaft **13** terminates in an anvil structure **16** which, in the illustrated embodiment, is a substantially open, or split, structure which is adapted for transverse movement to close about a portion of the horn. When the two halves of the split anvil structure **16** are brought together they form a radiused conforming surface **17** similar to the radiused conforming surface **14** on the horn, and like the horn can employ a number of geometries to maximize contact surface area. Also, like horn conforming surface **14,** anvil conforming surface **17** is provided with a protruding edge **18.** In other embodiments, where severing the loose ends of the suture is not required or where the ends are trimmed manually, protruding edges **15** and **18** are absent. In the preferred embodiment, there exists a relative curvature of the radiused conforming surfaces **14** and **17** in the plane of the axis of the radius of conforming surfaces **14** and **17.** On one preferred embodiment (shown) split anvil conforming surface **17** is convex relative to the space between the horn and the anvil **16,** while the horn conforming surface **14** is straight. In other embodiments the anvil surface is straight and the horn convex, in still others both are convex but to a lesser degree, others still show the horn concave and the anvil convex but to a greater degree than the concavity of the horn, yet another embodiment is a reverse of the latter. These preceding embodiments can be generalized as having the space **19** between conforming surfaces **14** and **17** exhibit a relative curvature that is convex as viewed from inside the space **19.**

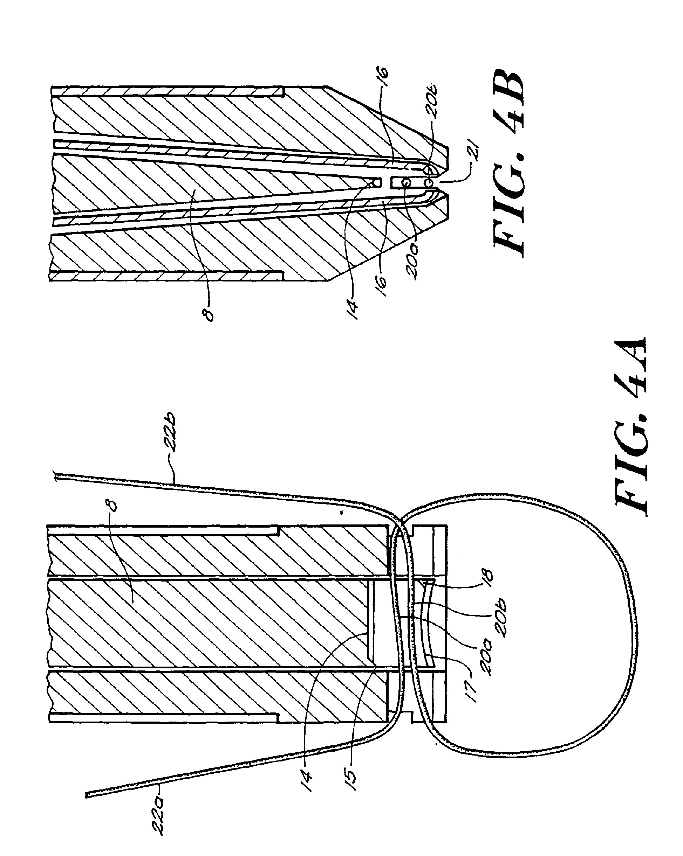

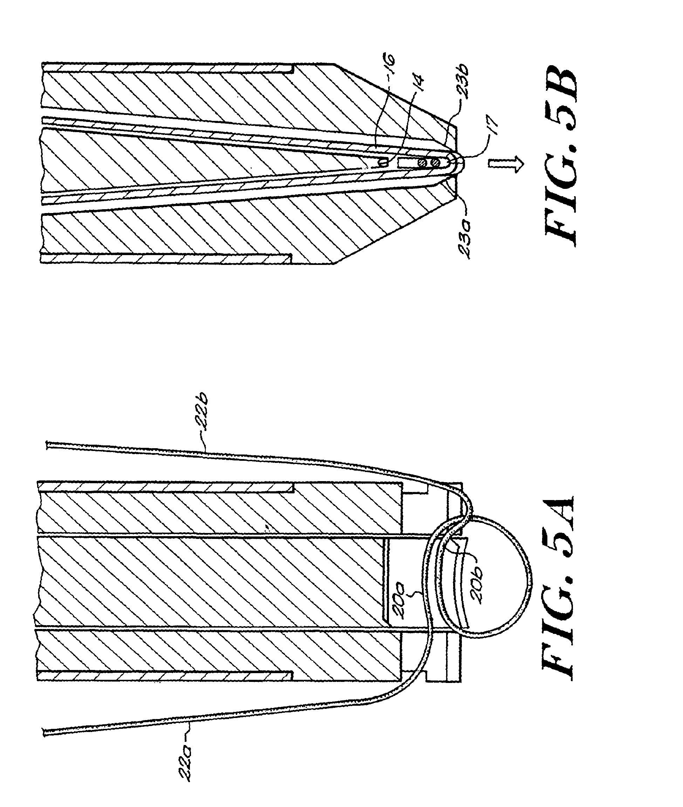

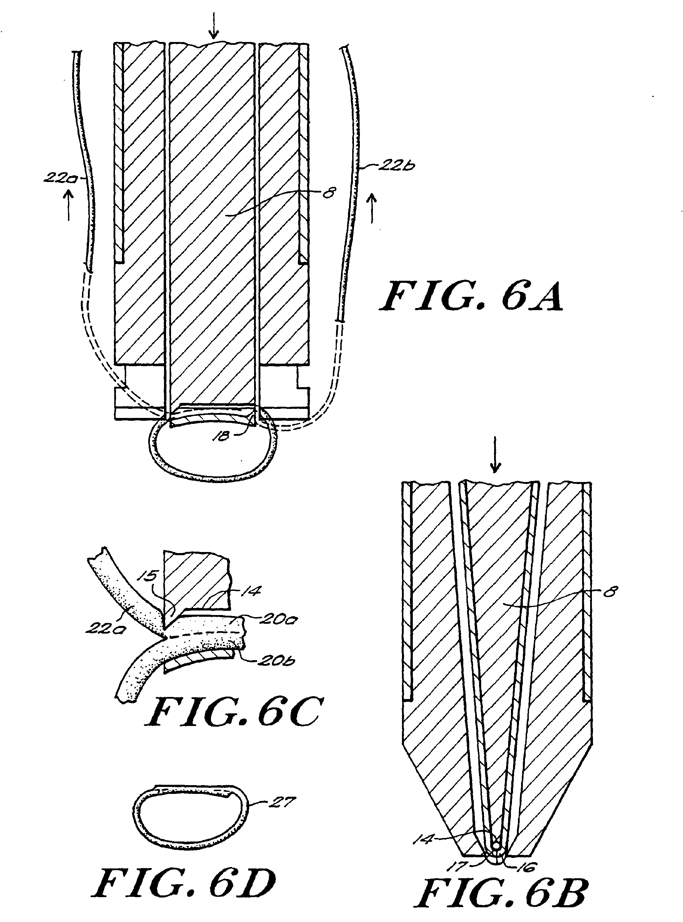

The cooperation of the horn **8** and split anvil **16** in the process of forming a welded suture can be seen in Figs. 4, 5 and 6. Fig. 4a is a cross section of slotted tip **4** in the plane of the axis of the radius of conforming surfaces **14** and **17** shown with the split anvil structure **16** separated to accept overlapping suture strands **20a** and **20b.** Fig. 4b is a section view of slotted tip **4** perpendicular to the plane of the axis of the radius of conforming surfaces **14** and **17.** In a preferred embodiment, slot **21** in slotted tip **4** has a width equal to or greater than the diameter of the overlapping suture strands **20a** and **20b,** but less than 2 diameters. In this way overlapping suture strands **20a** and **20b** can enter the slot only if parallel and not crossed. In other preferred embodiments slot **21** is slightly narrower than the diameter of suture strands **20a** and **20b** in order to create drag on the exiting strands **22a** and **22b** during tensioning and to hold them in place during welding. Figs. 5a and 5b show split anvil **16** approximated, thereby completing formation of anvil conforming surface **17.** Approximation of split anvil **16** is performed by displacing tubular inner shaft **13** which terminates in split anvil structure **16** distally relative to split tip 4, and in so doing, the opposed segments of split anvil structure **16,** which are biased to spring apart, glide on ramp surfaces **23a** and **23b** to move toward each other. In one preferred embodiment, opening and closing of the split anvil structure **16** is controlled by operation of actuator 5. Other embodiments employ other actuating means known to the art to accomplish this action. Once split anvil structure **16** is approximated, overlapping suture strands **20a** and **20b** are captive in slot **21** and can be tensioned by pulling on exiting strands **22a** and/or **22b.** Figs 6a and 6b show overlapping suture strands **20a** and **20b** being welded together. Horn **8** is displaced distally relative to anvil structure **16.** In a preferred embodiment, actuator **5** is exercised to displace housing **9** until horn conforming surface **14** contacts overlapping suture strands **20a** and **20b** arresting the motion of horn **8** and transducer **7** causing transducer **7** to compress spring **11** thereby compressing overlapping suture strands **20a** and **20b** against each other and against conforming surfaces **14** and **17** with a predetermined force supplied by spring **11.** Ultrasonic transducer **7** is activated causing horn conforming surface **14** to vibrate. Suture strand **20a,** being acoustically coupled with large contact area of horn conforming surface **14,** vibrates in sympathy with horn **8.** Suture strand **20b,** being acoustically coupled with large contact area of anvil conforming surface **17,** is non-vibrating. Suture strands **20a** and **20b,** being coupled respectively to vibrating and non-vibrating structures, vibrate against each other and, being substantially round in cross section, concentrate ultrasonic energy along the thin line or point where they contact each other. This concentration of energy where suture strands **20a** and **20b** make contact results in a buildup of heat, ultimately resulting in localized melting of the suture. Experience has shown that superior weld strength is achieved when a portion of the suture cross section on either side of the weld is not sacrificed to melting and thereby allowed to retain its original, highly linearized molecular orientation. Further, the amount of oriented material on either side of the weld must be controlled precisely to achieve optimum weld strength. A preferred way to control the amount of cross section sacrificed to welding is to control the amount of ultrasonic energy input to the overlapping suture strands **20a** and **20b.** The preferred embodiment includes means for measuring a characteristic of either the welded joint or the welding structure itself so as to control the energy delivered to the welding structure. Such characteristics include, for example, distance of travel of the horn, time elapsed after actuation of the horn and anvil, and force exerted on the segments by the horn. In one preferred embodiment, the change in displacement of the horn **8** relative to the split anvil structure **16** during the welding process is measured. Ultrasonic energy from the power supply is switched off when the displacement, which is proportional to the volume of material melted, which in turn is proportional to the energy input to the overlapping suture strands **20a** and **20b,** reaches a predetermined value. In the preferred embodiment the horn **8** maintains a predetermined compressive force on the overlapping suture strands **20a** and **20b** during the cooling and re-solidification process. This force can be measured and used to control the delivery of energy to the welding horn. Another preferred embodiment provides an electrical contact that is made or broken at a predetermined displacement of the horn **8** relative to the split anvil structure **16** during the welding process and in so doing switches off the ultrasonic energy at the appropriate energy input. In another embodiment, the time elapsed after actuation of the horn and anvil is measured by a clock, and delivery of energy to the welding horn is controlled by interrupting electrical power from the power supply after a predetermined time has elapsed. Other embodiments include controlling energy input directly through electronic measurement and control of power and time. However, this method does not account for ultrasonic energy lost to environmental conditions (particularly the presence of blood and other materials in the weld area) and other variables. Another embodiment provides a physical stop to limit the travel of the horn **8** to a predetermined distance. This embodiment has the disadvantage of disengaging the compressive force applied to the overlapping suture strands 20a and 20b during the cooling and re-solidification process, and thereby affecting the morphology of the weld region of the finished weld.

Other anvil structures are considered to be within the scope of the present invention. Although the anvil structure 16 is illustrated as being substantially symmetrically split, it may also be asymmetrically split, or be otherwise constructed to partially or fully surround the segments to be welded and at least a portion of the horn structure.

In a preferred embodiment means for severing the exiting strands 22a and 22b are provided, as seen in Fig. 6a and 6c. In one preferred embodiment protruding edges 15 and 18 are present on conforming surfaces 14 and 17. During welding, protruding edges 15 and 18 defeat acoustic coupling of conforming surfaces 14 and 17 at specific locations on the overlapping suture strands 20a and 20b corresponding to the point where exiting strands 22a and 22b connect to overlapping suture strands 20a and 20b at the edges of the weld region. By defeating acoustic coupling at protruding edge 15, overlapping suture strands 20a and 20b preferentially couple with split anvil structure 16, resulting in energy concentration at the interface of protruding edge 15 and suture strand 20a. The result is localized melting at this interface. In a preferred embodiment, the length of protruding edge 15 is sufficient to cause melting through most of the cross section of strand 20a, such that it is still attached to the exiting strand 22a but able to be separated from strand 20a by a gentle tug on exiting strand 22a. The preceding scenario is duplicated where overlapping suture strands 20a and 20b preferentially couple with horn 8 in the vicinity of protruding edge 18, resulting in near complete severing of the interface of overlapping strand 20b and exiting strand 22b. In other preferred embodiments the strands are severed completely. In still other embodiments the strands are left fully intact for manual trimming or to allow the exiting strands to be used for further tissue approximation, wound closure, ligating or suture anchoring functions.

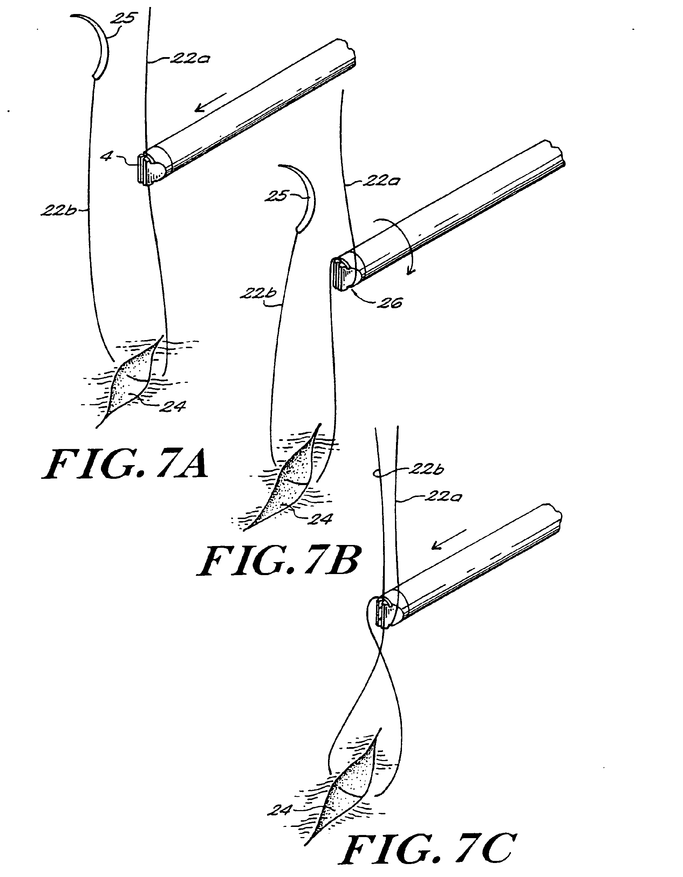

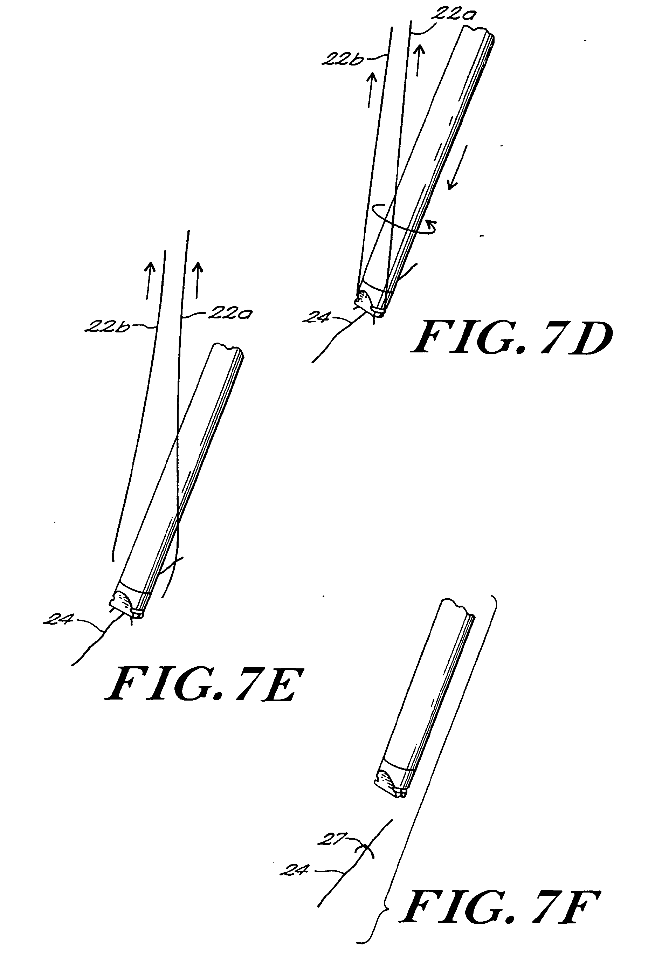

In the preferred embodiment the device is operated as shown in Figs. 7a through 7f. Fig. 7a shows a wound 24 to be closed by suture. A suture needle 25 is passed through either side of the wound leaving exiting strands **22a** and **22b.** With split anvil structure **16** in the open position, strand **22a** is slid into slot **21** in slotted tip **4.** Fig. 7b shows the device **1** rotated 180 degrees. Strand **22a** is prevented slipping free from tip **4** by circumferential retaining slot **26.** Fig. 7c shows strand **22b** slid into slot **21.** At this point split anvil structure **16** can be approximated by exercising actuator 5 on handle portion **2,** thereby capturing strands **22a** and **22b** and positioning overlapping suture strands **20a** and **20b.** Fig. 7d shows exiting strands **22a** and **22b** held under tension while slotted tip **4** is slid into close proximity with wound **24** holding the edges of the wound **24** closed. Fig. 7e shows the welding/cutting step where overlapping suture strands **20a** and **20b** are joined and exiting strands **22a** and **22b** are trimmed free. Fig. 7f shows the completed stitch **27** released from the instrument by opening split anvil structure **16.** In other embodiments of the device and method of surgery suture is used to surround a vessel (e. g. ligation, etc.), attach or suspend an anatomical or foreign structure (e. g. bladder neck suspension, mesh or film attachment, grafting, anastomosis, etc.) or create other stitch forms (e. g. subcutaneous, mattress, anchor for running stitch, etc.).

The invention may be embodied in other specific forms without departing from the spirit or essential characteristics thereof. The present embodiments are therefore to be considered in all respects as illustrative and not restrictive, the scope of the invention being indicated by the appended claims rather than by the foregoing description. All changes that come within the meaning of the claims are therefore intended to be embraced therein.

## Claims

1. A device for forming a welded joint in an elongated surgical suture material, said device comprising:
a handle portion (2),
an elongated tubular shaft portion (3) extending along an axis from said handle portion to a distal end;
a welding structure at the distal end of said shaft, said welding structure including a channel open to the distal end and extending transverse to the axis for accepting two or more segments of said elongated material therein, said segments being overlapped in the channel in the direction of the axis in a joint region, said welding structure further including a contoured horn (8) movable axially within said shaft, and a contoured anvil (16) movable axially within said shaft and relative to said horn, said anvil at least partially surrounding said horn;
means (5) for actuating said horn and anvil for relative movement to effect a welded joint in said segments of said elongated material; and
means for providing energy to the welding structure.

2. A device according to claim 1, wherein the anvil is a split structure adapted for transverse movement relative to said horn so as to effect closure of the anvil about a portion of the horn.

3. A device according to claim 1, wherein the segments are overlapped so that they share a common directionality in the joint region.

4. A device according to claim 1, wherein at least one of the horn and the anvil is contoured for maximum contact with the elongated material.

5. A device according to claim 1, further comprising a severing element on at least one of the horn and the anvil, whereby the severing element is adapted to cause localized melting of a suture segment in proximity thereto.

6. A device according to claim 1, wherein the means for actuating the horn and anvil for movement relative to one another includes trigger means on the handle and connected to the horn and anvil, first spring means disposed within the handle and coupled with the horn for biasing the horn toward the distal end of the shaft, and second spring means disposed within the tubular shaft for biasing the anvil away from the distal end of the shaft.

7. A device according to claim 6, further comprising means for detecting a characteristic of at least one of the welding structure and the welded joint after actuation of the horn and anvil, and means for controlling the delivery of energy to the welding structure when the characteristic reaches a predetermined value, and optionally, either:
a) wherein the means for controlling the delivery of energy to the welding structure comprises a switch for making or breaking an electrical circuit when the characteristic reaches a predetermined value;
b) wherein the characteristic is displacement of the horn relative to the anvil after actuation of the horn and anvil;
c) wherein the characteristic is time elapsed after actuation of the horn and anvil; or
d) wherein the characteristic is force exerted by the horn on the segments after actuation of the horn and anvil and during welding of the segments.

8. A device according to claim 1, wherein the means for providing a source of energy comprises an ultrasonic signal generator and an ultrasonic transducer coupled to the horn of the welding structure.

9. A device according to claim 1 and either, wherein the distal end of at least the welding horn includes an arcuate contour for defining the contact area between the welding horn and a segment in contact therewith, or
wherein the device further includes an exterior housing disposed about the shaft and the welding anvil, in which case, optionally,
wherein the welding anvil includes two cantilevered fingers having distal ends which define a base of the open channel, and
wherein the housing is adapted for sliding motion relative to the welding anvil, wherein the fingers are resiliently biased apart, and wherein axially directed sliding motion of the housing over the fingers toward their distal ends compresses the fingers together to form a closed channel.

## Patentansprüche

1. Vorrichtung zur Bildung einer Schweißverbindung in einem gestreckten chirurgischen Nahtmaterial, wobei diese Vorrichtung umfasst:
einen Griffabschnitt (2);
einen länglichen rohrförmigen Schaftabschnitt (3), der sich entlang einer Achse von diesem Griffabschnitt bis zu einem distalen Ende erstreckt;
eine Schweißanordnung am distalen Ende dieses Schafts, wobei diese Schweißanordnung einen Kanal beinhaltet, der zum distalen Ende hin offen ist und sich quer zur Achse erstreckt, um zwei oder mehr Segmente dieses gestreckten Materials darin aufzunehmen, wobei diese Segmente in dem Kanal in Richtung der Achse in einer Verbindungsregion überlappt sind, wobei diese Schweißanordnung ferner ein Horn mit einer Kontur (8) umfasst, das in diesem Schaft axial beweglich ist, und einen Amboss mit einer Kontur (16), der axial in diesem Schaft und relativ zu diesem Horn beweglich ist, wobei dieser Amboss dieses Horn wenigstens teilweise umgibt;
Mittel (5), um dieses Horn und diesen Amboss für eine relative Bewegung zu betätigen, um eine Schweißverbindung in diesen Segmenten dieses gestreckten Materials herbeizuführen; und
Mittel, um dieser Schweißanordnung Energie zu liefern.

2. Vorrichtung nach Anspruch 1, wobei dieser Amboss eine Spaltstruktur ist, die für eine Querbewegung relativ zu diesem Horn geeignet ist, um das Schließen des Ambosses über einen Teil des Horns zu bewirken.

3. Vorrichtung nach Anspruch 1, wobei die Segmente überlappt sind, so dass sie sich eine gemeinsame Bündelung in dem Verbindungsbereich teilen.

4. Vorrichtung nach Anspruch 1, wobei wenigstens eines aus Horn und Amboss eine Kontur für maximalen Kontakt mit dem gestreckten Material aufweist.

5. Vorrichtung nach Anspruch 1, ferner umfassend ein Trennelement auf wenigstens einem aus Horn und Amboss, wobei das Trennelement geeignet ist, um lokalisiertes Schmelzen eines Nahtsegments in seiner Nähe zu bewirken.

6. Vorrichtung nach Anspruch 1, wobei die Mittel zur Betätigung des Horns und des Ambosses relativ zueinander Mittel zum Auslösen auf dem Griff und verbunden mit dem Horn und dem Amboss, erste Federmittel, die in dem Griff angebracht und mit dem Horn verbunden sind, um das Horn gegenüber dem distalen Ende des Schafts vorzuspannen, und zweite Federmittel, die in dem rohrförmigen Schaft angebracht sind, um den Amboss vom distalen Ende des Schafts weg vorzuspannen, umfassen.

7. Vorrichtung nach Anspruch 6, ferner umfassend Mittel zur Detektion einer Kenngröße von wenigstens einem aus Schweißanordnung und Schweißverbindung nach der Betätigung des Horns und des Ambosses, und Mittel zur Steuerung der Abgabe von Energie an die Schweißanordnung, wenn die Kenngröße einen vorbestimmten Wert erreicht, und gegebenenfalls entweder:
a) wobei das Mittel zur Steuerung der Abgabe von Energie an die Schweißanordnung einen Schalter umfasst, um einen elektronischen Schaltkreis herzustellen oder zu unterbrechen, wenn die Kenngröße einen vorbestimmten Wert erreicht;
b) wobei die Kenngröße die Verschiebung des Horns relativ zu dem Amboss nach der Betätigung des Horns und des Ambosses ist;
c) wobei die Kenngröße die Zeit ist, die nach der Betätigung des Horns und des Ambosses vergangen ist; oder
d) wobei die Kenngröße die Kraft ist, die von dem Horn auf die Segmente nach der Betätigung des Horns und des Ambosses und während des Schweißens der Segmente ausgeübt wird.

8. Vorrichtung nach Anspruch 1, wobei das Mittel zur Bereitstellung einer Energiequelle einen Generator für Ultraschallsignale und einen Ultraschallumwandler, verbunden mit dem Horn der Schweißanordnung, umfasst,

9. Vorrichtung nach Anspruch 1, und wobei entweder das distale Ende von wenigstens dem Schweißhorn eine gebogene Kontur umfasst, um den Kontaktbereich zwischen dem Schweißhorn und einem dazu in Kontakt stehenden Segment festzulegen, oder wobei die Vorrichtung ferner ein Außengehäuse umfasst, das um den Schaft und den Schweißamboss herum angebracht ist, wobei in diesem Fall gegebenenfalls der Schweißamboss zwei freitragende Finger umfasst, die distale Enden aufweisen, die eine Basis des offenen Kanals definieren, und wobei das Gehäuse für eine Gleitbewegung relativ zu dem Schweißamboss geeignet ist, wobei die Finger elastisch vorgespannt sind, und wobei die axial gerichtete Gleitbewegung des Gehäuses über die Finger zu ihren distalen Enden hin die Finger zusammendrückt, um einen geschlossenen Kanal zu bilden.

## Revendications

1. Dispositif pour former une liaison soudée dans un matériau de suture chirurgicale allongé, ledit dispositif comprenant :
une portion de poignée (2) ;
une portion de tige tubulaire allongée (3) s'étendant le long d'un axe à partir de ladite portion de poignée vers une extrémité distale ;
une structure de soudage au niveau de l'extrémité distale de ladite tige, ladite structure de soudage comprenant un canal ouvert vers l'extrémité distale et s'étendant de manière transversale par rapport à l'axe pour recevoir deux segments ou plus dudit matériau allongé dans celui-ci, lesdits segments étant superposés dans le canal dans la direction de l'axe dans une région de liaison, ladite structure de soudage comprenant en outre un cornet profilé (8) pouvant se déplacer axialement dans ladite tige, et une enclume profilée (16) pouvant se déplacer axialement dans ladite tige et par rapport audit cornet, ladite enclume entourant au moins partiellement ledit cornet ;
des moyens (5) pour actionner lesdits cornet et enclume pour qu'un mouvement relatif réalise une liaison soudée dans lesdits segments dudit matériau allongé ; et
des moyens pour fournir de l'énergie à la structure de soudage.

2. Dispositif selon la revendication 1, dans lequel l'enclume est une structure fendue adaptée pour un mouvement transversal par rapport audit cornet de manière à réaliser une fermeture de l'enclume autour d'une portion du cornet.

3. Dispositif selon la revendication 1, dans lequel les segments se superposent de sorte qu'ils partagent une orientation commune dans la région de liaison

4. Dispositif selon la revendication 1, dans lequel au moins un des cornet et lenclume est façonné pour un contact maximum avec le matériau allongé.

5. Dispositif selon la revendication 1, comprenant en outre un élément de coupe sur au moins un des cornet et enclume, moyennant quoi l'élément de coupe est adapté pour causer une fusion localisée d'un segment de suture à proximité de celui-ci.

6. Dispositif selon la revendication 1, dans lequel le moyen pour actionner le cornet et l'enclume dans un mouvement l'un par rapport à l'autre comprend des moyens de déclenchement sur la poignée et connectés au cornet et à l'enclume, des premiers moyens à ressort disposés dans la poignée et couplés avec le cornet pour rapprocher le cornet de l'extrémité distale de la tige, et des seconds moyens à ressort disposés dans la tige tubulaire pour éloigner l'enclume de l'extrémité distale de la tige.

7. Dispositif selon la revendication 6, comprenant en outre des moyens pour détecter une caractéristique d'au moins une des structure de soudage et de la liaison soudée après l'actionnement du cornet et de l'enclume, et des moyens pour commander la distribution d'énergie à la structure de soudage quand la caractéristique atteint une valeur prédéterminée, et, en option, soit :
a) dans lequel les moyens pour contrôler la distribution d'énergie à la structure de soudage comprennent un commutateur pour fermer ou ouvrir un circuit électrique quand la caractéristique atteint une valeur prédétermine ;
b) dans lequel la caractéristique est un déplacement du cornet par rapport à l'enclume après l'actionnement du cornet et de l'enclume ;
c) dans lequel la caractéristique est le temps écoulé après l'actionnement du cornet et de l'enclume ; ou
d) où la caractéristique est une force exercée par le cornet sur les segments après actionnement du cornet et de l'enclume et pendant le soudage des segments.

8. Dispositif selon la revendication 1, dans lequel les moyens pour fournir une source d'énergie comprennent un générateur de signal ultrasonore et un transducteur ultrasonore couplés au cornet de la structure de soudage.

9. Dispositif selon la revendication 1 dans lequel soit l'extrémité distale au moins du cornet de soudage comprend un profil arqué pour définir la zone de contact entre le cornet de soudage et un segment en contact avec celle-ci; soit le dispositif comprend en outre un boîtier extérieur disposé autour de la tige et l'enclume de soudage, auquel cas, en option, l'enclume de soudage comprend des doigts en porte-à-faux dont des extrémités distales définissent une base du canal ouvert, et dans lequel le boîtier est adapté pour un mouvement coulissant par rapport à l'enclume de soudage, dans lequel les doigts sont écartés l'un de l'autre par une sollicitation élastique, et dans lequel le mouvement de coulissage axial du boîtier sur les doigts en direction de leurs extrémités distales comprime les doigts ensemble pour former un canal fermé.
